# EUROPEAN PATENT SPECIFICATION

(11) **EP 4 520 969 B1**
(45) Date of publication and mention of the grant of the patent: **04.11.2026**
(21) Application number: 24198383.2
(22) Date of filing: 04.09.2024
(51) Int. Cl.: F04B 37/14, F04B 39/02, F04B 49/06, F04B 53/18, F04C 25/02, F04C 28/28, F04C 29/02, G01N 21/27, G01N 21/31, G01N 33/28

(54) **LUBRICATING-OIL MONITORING SYSTEM AND VACUUM PUMP SYSTEM**
SCHMIERÖLÜBERWACHUNGSSYSTEM UND VAKUUMPUMPENSYSTEM
SYSTÈME DE SURVEILLANCE D'HUILE DE LUBRIFICATION ET SYSTÈME DE POMPE À VIDE

(30) Priority: 07.09.2023 JP 2023144947
(43) Date of publication of application: 12.03.2025
(73) Proprietor: EBARA CORPORATION, Ota-ku, Tokyo 144-8510 (JP)
(72) Inventor: NAKAMURA, Yumiko, Tokyo, 1448510 (JP); KON, Toshiya, Tokyo, 1448510 (JP)
(74) Representative: Carstens, Dirk Wilhelm

(56) References cited:
- EP-A1- 3 822 560
- WO-A1-2010/150526
- AU-A1- 2013 278 311
- JP-A- 2011 094 761
- JP-A- 2019 045 166
- US-A1- 2021 372 420

## Description

### BACKGROUND OF THE INVENTION

### Field of the Invention:

The present invention relates to a lubricating-oil monitoring system for monitoring a condition of a lubricating oil supplied to a bearing of a vacuum pump and a condition of the bearing, and a vacuum pump system including the lubricating-oil monitoring system. Description of the Related Art:

In process of manufacturing semiconductor devices, liquid crystal panels, LEDs, solar cells, etc., a process gas is introduced into a process chamber to perform a certain type of process, such as etching process or CVD process. The process gas that has been introduced into the process chamber is exhausted by a vacuum pump. Generally, the vacuum pump used in these manufacturing processes that require high cleanliness is so-called dry vacuum pump that does not use oil in gas flow passages. One typical example of such a dry vacuum pump is a positive-displacement vacuum pump having a pair of pump rotors in a pump chamber which are rotated in opposite directions to deliver the gas.

In the vacuum pump, lubricating oil is used to lubricate and cool a bearing configured to rotatably support a rotation shaft to which a pump rotor is secured. The vacuum pump includes a lubricating-oil chamber holding the lubricating oil. When the vacuum pump is in motion, the lubricating oil in the lubricating-oil chamber is supplied to the bearing, lubricates and cools the bearing, and is then returned to the lubricating-oil chamber.

### Citation List

### Patent Literature

Patent document 1: International patent publication No. WO 2010-150526
Patent document 2: Japanese laid-open patent publication No. 2011-94761
Patent document 3: Japanese laid-open patent publication No. 2019-45166

In order to prevent breakdown of the vacuum pump due to an abnormality in the bearing, etc., maintenance (e.g., a replacement of the lubricating oil, a replacement of the bearing, or an overhaul of the vacuum pump) is performed periodically. Since the maintenance is performed based on an accumulated operating time of the vacuum pump, regardless of a condition of the vacuum pump, the maintenance may be performed even on the vacuum pump that is able to continue to operate. Stopping the operation of the vacuum pump that is able to continue to operate for the maintenance of the vacuum pump is inefficient from a viewpoint of cost and operation time. In addition, if the vacuum pump breaks down before the maintenance period is reached, the vacuum pump may be forced to make an emergency stop during operation of the vacuum pump, and as a result, loss of wafers or the like that are being processed may occur. Therefore, it is required to monitor the condition of the vacuum pump in order to perform the maintenance of the vacuum pump at an appropriate time.

### SUMMARY OF THE INVENTION

Thus, the present invention provides a lubricating-oil monitoring system capable of monitoring a condition of a vacuum pump in order to perform maintenance of the vacuum pump at an appropriate time, and a vacuum pump system including the lubricating-oil monitoring system.

Lubricating oil supplied to a bearing of the vacuum pump is normally colorless and transparent. However, if a substance produced by an abnormality, such as wear of the bearing, or foreign matter derived from a process gas, is mixed into the lubricating oil, a hue of the lubricating oil may change. The inventors have found that a condition of the lubricating oil and a condition of the bearing can be judged by optically measuring the lubricating oil.

Accordingly, in one embodiment, there is provided a lubricating-oil monitoring system comprising: a lubricating-oil measuring device configured to optically measure lubricating oil supplied to a bearing of a vacuum pump; and a data processor electrically coupled to the lubricating-oil measuring device, wherein the lubricating-oil measuring device includes: an oil inspection chamber into which the lubricating oil is introduced; a light source configured to irradiate the lubricating oil in the oil inspection chamber with inspection light containing red light, green light, and blue light; a first light receiving element configured to receive reflected light from the lubricating oil irradiated with the inspection light; and a second light receiving element configured to receive transmitted light from the lubricating oil irradiated with the inspection light, and the data processor is configured to judge a condition of the lubricating oil and a condition of the bearing based on a combination of a color of the reflected light and a color of the transmitted light.

In one embodiment, the data processor is configured to: resolve each of the reflected light and the transmitted light into a red component, a green component, and a blue component; and judge the condition of the lubricating oil and the condition of the bearing based on intensities of the red component, the green component, and the blue component of each of the reflected light and the transmitted light.

In one embodiment, the condition of the lubricating oil and the condition of the bearing include an abnormality in the bearing, contamination of the lubricating oil, and deterioration of the lubricating oil.

In one embodiment, the data processor is configured to judge that an abnormality has occurred in the bearing when the intensities of the red component, the green component, and the blue component of the reflected light are smaller than a predetermined bearing-abnormality threshold value and the intensities of the red component, the green component, and the blue component of the transmitted light are smaller than the predetermined bearing-abnormality threshold value.

In one embodiment, the data processor is configured to judge that the lubricating oil has been contaminated with foreign matter when the intensities of the red component, the green component, and the blue component of the reflected light are larger than a predetermined lubricating-oil-contamination threshold value and the intensities of the red component, the green component, and the blue component of the transmitted light are smaller than the predetermined lubricating-oil-contamination threshold value.

In one embodiment, the data processor is configured to judge that the lubricating oil has been deteriorated when the intensity of the red component of the reflected light is larger than a predetermined lubricating-oil-deterioration threshold value and the intensities of the green component and the blue component of the reflected light are smaller than the lubricating-oil-deterioration threshold value.

In one embodiment, the data processor is configured to: calculate a color difference of the reflected light with respect to a reflected reference color based on the intensities of the red component, the green component, and the blue component of the reflected light; calculate a color difference of the transmitted light with respect to a transmitted reference color based on the intensities of the red component, the green component, and the blue component of the transmitted light; and judge the condition of the lubricating oil and the condition of the bearing based on the color difference of the reflected light and the color difference of the transmitted light.

In one embodiment, the lubricating oil comprises a fluorine-based lubricating oil.

In one embodiment, there is provided a vacuum pump system comprising: a vacuum pump; and the above-mentioned lubricating-oil monitoring system, wherein the vacuum pump includes: the bearing; and a housing structure that forms a lubricating-oil chamber holding the lubricating oil, the lubricating-oil measuring device is attached to the housing structure, and the oil inspection chamber communicates with the lubricating-oil chamber.

In one embodiment, the vacuum pump system further comprises: a pump controller configured to control an operation of the vacuum pump, wherein the pump controller is configured to output an alarm signal when the data processor judges that an abnormality has occurred in the bearing, when the data processor judges that the lubricating oil has been contaminated with foreign matter, or when the data processor judges that the lubricating oil has been deteriorated.

According to the present invention, the lubricating-oil monitoring system is configured to judge the condition of the lubricating oil and the condition of the bearing based on the combination of the color of the reflected light and the color of the transmitted light. Therefore, the condition of the lubricating oil and the condition of the bearing can be monitored for maintenance of the vacuum pump at an appropriate time.

### BRIEF DESCRIPTION OF THE DRAWINGS

FIG. 1 is a schematic diagram showing an embodiment of a vacuum pump system;
FIG. 2 is a cross-sectional view of a vacuum pump shown in FIG. 1;
FIG. 3 is a cross-sectional view taken along line A-A in FIG. 1; and
FIG. 4 is a cross-sectional view taken along line B-B in FIG. 3.

### DESCRIPTION OF EMBODIMENTS

Embodiments will be described below with reference to the drawings.

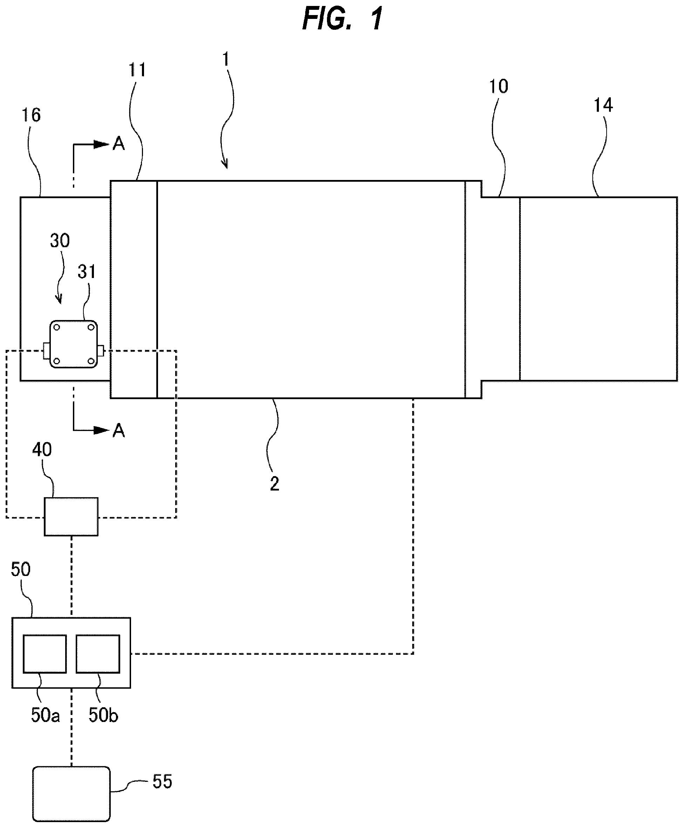

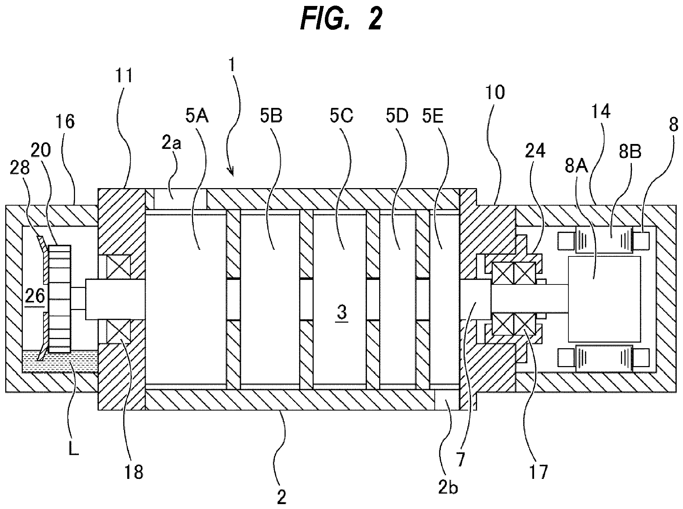

FIG. 1 is a schematic diagram showing an embodiment of a vacuum pump system, and FIG. 2 is a cross-sectional view of a vacuum pump 1 shown in FIG. 1. As shown in FIG. 1, the vacuum pump system includes the vacuum pump 1 configured to evacuate a process gas used in manufacturing of semiconductor devices, liquid crystals, LEDs, solar cells, or the like, and a lubricating-oil monitoring system 30 configured to monitor a condition of lubricating oil and a condition of a bearing used in the vacuum pump 1.

The vacuum pump 1 of the embodiment described below is a positive-displacement vacuum pump. In particular, the vacuum pump 1 shown in FIG. 1 is a so-called dry vacuum pump that does not use oil in its flow passages for a gas. Since a vaporized oil does not flow to an upstream side, the dry vacuum pump can be suitably used for a manufacturing apparatus for semiconductor devices or the like that requires high cleanliness.

As shown in FIG. 2, the vacuum pump 1 includes a pump casing 2 having a pump chamber 3 therein, pump rotors 5A to 5E arranged in the pump chamber 3, rotation shafts 7 to which the pump rotors 5A to 5E are secured, and electric motor 8 coupled to the rotation shaft 7. The pump rotors 5A to 5E and each rotation shaft 7 may be an integral structure. Although only one set of pump rotors 5A to 5E and only one rotation shaft 7 are depicted in FIG. 2, in this embodiment, a pair of pump rotors 5A to 5E are arranged in the pump chamber 3, and the pair of pump rotors 5A to 5E are secured to a pair of rotation shafts 7, respectively. The electric motor 8 is coupled to one of the pair of rotation shafts 7. In one embodiment, a pair of electric motors 8 may be coupled to the pair of rotation shafts 7, respectively.

The pump rotors 5A to 5E of the present embodiment are Roots-type pump rotors, while in one embodiment the pump rotors 5A to 5E may be claw-type pump rotors. Further, the pump rotors 5A to 5E may be a combination of Roots-type and claw-type pump rotors. Although the pump rotors 5A to 5E of the present embodiment are multi-stage pump rotors, in one embodiment the pump rotors may be single-stage pump rotors.

The vacuum pump 1 further includes side covers 10 and 11 located outwardly of the pump casing 2 in an axial direction of the rotation shafts 7. The side covers 10 and 11 are provided on both sides of the pump casing 2 and are coupled to the pump casing 2. In this embodiment, the side covers 10 and 11 are fixed to end surfaces of the pump casing 2 by not-shown screws. In one embodiment, the side covers 10, 11 and the pump casing 2 may be an integral structure.

The pump chamber 3 is formed by an inner surface of the pump casing 2 and inner surfaces of the side covers 10 and 11. The pump casing 2 has an intake port 2a and an exhaust port 2b. The intake port 2a is coupled to a chamber (not shown) filled with gas to be delivered. In one example, the intake port 2a may be coupled to a process chamber of a semiconductor-device manufacturing apparatus, and the vacuum pump 1 may be used for evacuating a process gas from the process chamber.

The vacuum pump 1 further includes a motor housing 14 and a gear housing 16 which are housing structures located outwardly of the side covers 10 and 11 in the axial direction of the rotation shafts 7. The side cover 10 is located between the pump casing 2 and the motor housing 14, and the side cover 11 is located between the pump casing 2 and the gear housing 16.

The motor housing 14 accommodates a motor rotor 8A and a motor stator 8B of the electric motor 8 therein. Inside the gear housing 16, a pair of gears 20 that mesh with each other are arranged. In FIG. 2, only one gear 20 is depicted. The electric motor 8 is rotated by a not-shown motor driver, and one rotation shaft 7 to which the electric motor 8 is coupled rotates the other rotation shaft 7 to which the electric motor 8 is not coupled in an opposite direction via the gears 20.

In one embodiment, a pair of electric motors 8, which are coupled to the pair of rotation shafts 7, respectively, may be provided. The pair of electric motors 8 are synchronously rotated in opposite directions by a not-shown motor driver, so that the pair of rotation shafts 7 and the pair of pump rotors 5A to 5E are synchronously rotated in opposite directions. In this case, the role of the gears 20 is to prevent loss of the synchronous rotation of the pump rotors 5 due to a sudden external cause.

In the embodiment shown in FIGS. 1 and 2, the motor housing 14 is arranged outwardly of the side cover 10, and the gear housing 16 is arranged outwardly of the side cover 11, while a configuration of the vacuum pump 1 is not limited to this embodiment. In one embodiment, the gear housing 16 may be arranged outwardly of the side cover 10, and the motor housing 14 may be arranged outwardly of the side cover 11. Further, in one embodiment, both the motor housing 14 and the gear housing 16 may be arranged outwardly of either the side cover 10 or the side cover 11.

When the pump rotors 5A to 5E are rotated by the electric motor 8, a gas is sucked into the pump chamber 3 of the pump casing 2 through the intake port 2a. The gas is sequentially compressed by the rotating pump rotors 5A to 5E, delivered to the exhaust port 2b, and discharged from the pump chamber 3 through the exhaust port 2b.

Each rotation shaft 7 is rotatably supported by bearings 17 and 18. The bearing 17 is held by a bearing housing 24, and the bearing 18 is supported by the side cover 11. The bearing 17 is coupled to the side cover 10 via the bearing housing 24. More specifically, the bearing housing 24 is held by the side cover 10. In one embodiment, a bearing housing holding the bearing 18 may be disposed between the side cover 11 and the bearing 18.

The vacuum pump 1 includes a housing structure that forms a lubricating-oil chamber holding lubricating oil for lubricating and cooling the bearings 17 and/or the bearings 18. In this embodiment, the housing structure is the gear housing 16, which forms a lubricating-oil chamber 26 holding lubricating oil L for lubricating and cooling the bearings 18. In one embodiment, the housing structure is the motor housing 14, which may form a lubricating-oil chamber holding lubricating oil for lubricating and cooling the bearings 17.

Oil plates 28 are attached to end surfaces of the gears 20 in the gear housing 16. The oil plates 28 are coupled to the pair of rotation shafts 7, respectively, and rotate together with the rotation shafts 7. In one embodiment, the oil plate 28 may be coupled to one of the pair of rotation shafts 7. The lubricating oil L is splashed up by the rotation of the oil plates 28 onto the bearings 18, so that the lubricating oil L is supplied to the bearings 18. The lubricating oil L supplied to the bearings 18 lubricates and cools the bearings 18, and is then returned to the lubricating-oil chamber 26. The lubricating oil L of the present embodiment is colorless and transparent. An example of the lubricating oil is a fluorine-based lubricating oil. However, the lubricating oil L is not limited to this embodiment, and may be lubricating oil having another color.

A hue of the lubricating oil L supplied to the bearings 18 of the vacuum pump 1 changes due to contamination by a substance produced by an abnormality, such as wear of the bearing 18, or foreign matter derived from the process gas. When wear particles due to the wear of the bearing 18 or fine debris produced by damage to the bearing 18 are mixed into the lubricating oil L, the color of the lubricating oil L changes to black.

When the foreign matter derived from the process gas is mixed into the lubricating oil L, the lubricating oil L may become cloudy. The foreign matter derived from the process gas is a by-product (e.g., SiO₂, NH₄Cl, etc.) of the process gas. In one example, when hard particles as the by-product of the process gas, such as SiO₂, are mixed into the lubricating oil L, the hard particles may accelerate the wear of the bearing 18. In another example, when corrosive particles as the by-product of the process gas, such as NH₄Cl, are mixed into the lubricating oil L, the corrosive particles may corrode the bearing 18. In addition, when properties of the lubricating oil L are changed and the lubricating oil L is deteriorated due to an influence of the process gas, the color of the lubricating oil L may change to red. Therefore, the lubricating-oil monitoring system 30 is configured to monitor a condition of the lubricating oil L and a condition of the bearing 18 by optically measuring the lubricating oil L.

As shown in FIG. 1, the lubricating-oil monitoring system 30 includes a lubricating-oil measuring device 31 configured to optically measure the lubricating oil L supplied to the bearings 18 of the vacuum pump 1, and a data processor 40 electrically coupled to the lubricating-oil measuring device 31. The lubricating-oil measuring device 31 is attached to an outer side-surface of the gear housing 16. The position of the lubricating-oil measuring device 31 is such that the lubricating-oil measuring device 31 faces an oil level of the lubricating oil L in the gear housing 16. In one embodiment, the lubricating-oil measuring device 31 may be attached to an end surface of the gear housing 16.

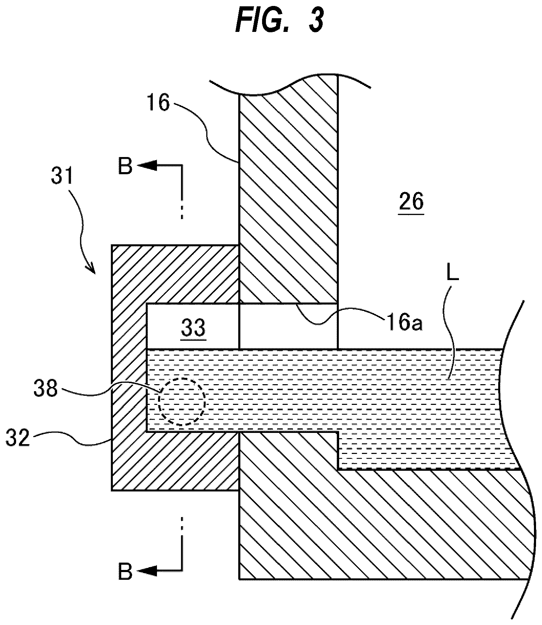

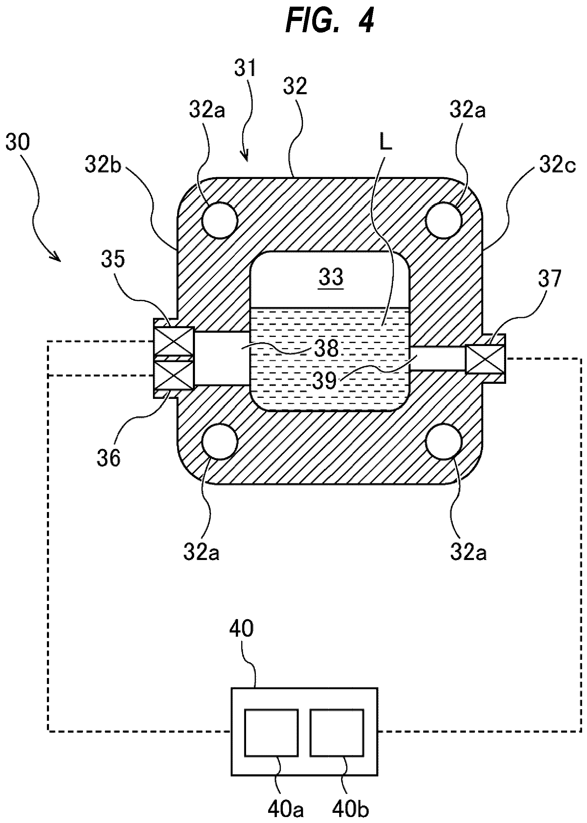

FIG. 3 is a cross-sectional view taken along line A-A in FIG. 1, and FIG. 4 is a cross-sectional view taken along line B-B in FIG. 3. As shown in FIG. 3, a side wall of the gear housing 16 has a through-hole 16a, and the lubricating-oil measuring device 31 is attached to this side wall of the gear housing 16. The through-hole 16a is formed near a bottom of the gear housing 16, and a lower end of the through-hole 16a is located lower than the oil level of the lubricating oil L in the lubricating-oil chamber 26. Therefore, the lubricating oil L in the lubricating-oil chamber 26 flows out to an outside of the gear housing 16 through the through-hole 16a. The lubricating-oil measuring device 31 is attached so as to face the through-hole 16a of the gear housing 16.

The lubricating-oil measuring device 31 includes a body case 32, and an oil inspection chamber 33 into which the lubricating oil L in the lubricating-oil chamber 26 is introduced. The oil inspection chamber 33 is formed by an inner surface of the body case 32. The oil inspection chamber 33 is open in a contact surface of the body case 32 with the gear housing 16, so that the oil inspection chamber 33 communicates with the lubricating-oil chamber 26 through the through-hole 16a. The lubricating oil L in the lubricating-oil chamber 26 is introduced into the oil inspection chamber 33 through the through-hole 16a.

As shown in FIG. 4, the body case 32 has four through-holes 32a near corners of the body case 32. Fastening tools (e.g., screws) for fixing the lubricating-oil measuring device 31 to the gear housing 16 are inserted into the through-holes 32a. It is noted that the number and positions of the through-holes 32a are not particularly limited as long as the lubricating-oil measuring device 31 can be fixed to the gear housing 16.

The lubricating-oil measuring device 31 includes a light source 35 configured to irradiate the lubricating oil L in the oil inspection chamber 33 with inspection light, a first light receiving element 36 configured to receive reflected light from the lubricating oil L irradiated with the inspection light, and a second light receiving element 37 configured to receive transmitted light from the lubricating oil L irradiated with the inspection light. The light source 35 and the first light receiving element 36 are attached to one side wall 32b of the body case 32. The second light receiving element 37 is attached to the other side wall 32c of the body case 32. The oil inspection chamber 33 is located between the light source 35 and the second light receiving element 37, and is located between the first light receiving element 36 and the second light receiving element 37. The inspection light emitted from the light source 35 contains red light, green light, and blue light.

The body case 32 has a first light transmissive portion 38 adjacent to the light source 35 and the first light receiving element 36, and further has a second light transmissive portion 39 adjacent to the second light receiving element 37. The first light transmissive portion 38 is located in the one side wall 32b of the body case 32, and extends from the light source 35 and the first light receiving element 36 to the oil inspection chamber 33. The second light transmissive portion 39 is located in the other side wall 32c of the body case 32, and extends from the second light receiving element 37 to the oil inspection chamber 33.

The first light transmissive portion 38 and the second light transmissive portion 39 are made of a transparent material through which the inspection light emitted from the light source 35, the reflected light from the lubricating oil L, and the transmitted light from the lubricating oil L can pass. However, the configurations of the first light transmissive portion 38 and the second light transmissive portion 39 are not particularly limited as long as the first light transmissive portion 38 and the second light transmissive portion 39 can allow the inspection light emitted from the light source 35, the reflected light from the lubricating oil L, and the transmitted light from the lubricating oil L to pass therethrough. For example, in one embodiment, the entire body case 32 may be made of a transparent material that can allow the transmitted light to pass through the body case 32.

The first light transmissive portion 38 and the second light transmissive portion 39 are arranged at a position lower than the oil level of the lubricating oil L in the oil inspection chamber 33. The first light transmissive portion 38 is located on an optical path of the inspection light emitted from the light source 35 and an optical path of the reflected light from the lubricating oil L. The second light transmissive portion 39 is located on an optical path of the transmitted light from the lubricating oil L.

The inspection light emitted from the light source 35 is directed to the lubricating oil L in the oil inspection chamber 33 through the first light transmissive portion 38. The inspection light directed to the lubricating oil L is reflected by a surface of the lubricating oil L, while passing through the lubricating oil L to the second light transmissive portion 39. A straight line interconnecting the first light transmissive portion 38 and the second light transmissive portion 39 is located on the optical path of the light passing through the lubricating oil L. The reflected light from the lubricating oil L irradiated with the inspection light is received by the first light receiving element 36 through the first light transmissive portion 38. The transmitted light from the lubricating oil L irradiated with the inspection light is received by the second light receiving element 37 through the second light transmissive portion 39.

The light source 35, the first light receiving element 36, and the second light receiving element 37 are electrically coupled to the data processor 40. The data processor 40 is configured to instruct the light source 35 to irradiate the lubricating oil L in the oil inspection chamber 33 with the light. Measurement data of the reflected light from the lubricating oil L received by the first light receiving element 36 and measurement data of the transmitted light from the lubricating oil L received by the second light receiving element 37 are transmitted to the data processor 40. RGB color sensors can be used for the first light receiving element 36 and the second light receiving element 37. The measurement data of the reflected light from the lubricating oil L received by the first light receiving element 36 includes color data of the reflected light. The measurement data of the transmitted light from the lubricating oil L received by the second light receiving element 37 includes color data of the transmitted light.

The data processor 40 is configured to judge the condition of the lubricating oil L and the condition of the bearing 18 based on a combination of the color of the reflected light from the lubricating oil L received by the first light receiving element 36 and the color of the transmitted light from the lubricating oil L received by the second light receiving element 37. More specifically, the data processor 40 resolves the reflected light into a red component, a green component, and a blue component based on the measurement data of the reflected light transmitted from the first light receiving element 36, and resolves the transmitted light into a red component, a green component, and a blue component based on the measurement data of the transmitted light transmitted from the second light receiving element 37. Further, the data processor 40 judges the condition of the lubricating oil L and the condition of the bearing 18 based on intensities of the red component, the green component, and the blue component of each of the reflected light and the transmitted light. Specific examples of the condition of the lubricating oil L and the condition of the bearing 18 include an abnormality in the bearing 18, contamination of the lubricating oil L, and deterioration of the lubricating oil L.

If a substance produced due to an abnormality in the bearing 18 is mixed into the lubricating oil L, the color of the lubricating oil L changes to black. As a result, the intensities of the red component, the green component, and the blue component of the reflected light from the lubricating oil L, and the intensities of the red component, the green component, and the blue component of the transmitted light from the lubricating oil L become smaller than those of normal (new) lubricating oil. Thus, the data processor 40 judges that an abnormality has occurred in the bearing 18 when the intensities of the red component, the green component, and the blue component of the reflected light are smaller than a predetermined bearing-abnormality threshold value and the intensities of the red component, the green component, and the blue component of the transmitted light are smaller than the predetermined bearing-abnormality threshold value. The intensities of the red component, the green component, and the blue component are represented by, for example, numerical values ranging from 0 to 255.

If foreign matter derived from the process gas is mixed into the lubricating oil L, the lubricating oil L becomes cloudy. As a result, the intensities of the red component, the green component, and the blue component of the reflected light from the lubricating oil L are larger than those of normal (new) lubricating oil, while the intensities of the red component, the green component, and the blue component of the transmitted light from the lubricating oil L are smaller than those of the normal (new) lubricating oil. Thus, the data processor 40 judges that the lubricating oil L has been contaminated with foreign matter when the intensities of the red component, the green component, and the blue component of the reflected light are larger than a predetermined lubricating-oil-contamination threshold value and the intensities of the red component, the green component, and the blue component of the transmitted light are smaller than the predetermined lubricating-oil-contamination threshold value.

The lubricating-oil-contamination threshold value is determined in advance based on a type of the process gas evacuated by the vacuum pump 1. A type of the foreign matter mixed into the lubricating oil L varies depending on the type of the process gas evacuated by the vacuum pump 1. For example, when the process gas contains silicon, SiO₂ particles which are a by-product of the process gas may be mixed into the lubricating oil L. The SiO₂ particles mixed into the lubricating oil L may accelerate wear of the bearing 18, so that there is a high risk of causing an abnormality in the bearing 18. Therefore, when the vacuum pump 1 evacuates the process gas containing the silicon, the lubricating-oil-contamination threshold value may be determined to be a relatively small value.

When the lubricating oil L is deteriorated (or is corroded) due to an influence of the process gas, the color of the lubricating oil L may change to red. In such a case, the intensity of the red component of the reflected light is larger than that of normal (new) lubricating oil, while the intensities of the green component and the blue component of the reflected light are smaller than those of the normal (new) lubricating oil L. Thus, the data processor 40 judges that the lubricating oil L has been deteriorated when the intensity of the red component of the reflected light is larger than a predetermined lubricating-oil-deterioration threshold value and the intensities of the green component and the blue component of the reflected light are smaller than the lubricating-oil-deterioration threshold value.

The data processor 40 is composed of at least one computer. The data processor 40 includes a memory 40a storing programs therein, and an arithmetic device 40b configured to perform arithmetic operations according to instructions contained in the programs. The memory 40a includes a main memory, such as a random access memory (RAM), and an auxiliary memory, such as a hard disk drive (HDD) or a solid state drive (SSD). Examples of the arithmetic device 40b include a CPU (central processing unit) and a GPU (graphic processing unit). However, the specific configurations of the data processor 40 are not limited to these examples.

According to this embodiment, the lubricating-oil monitoring system 30 is configured to judge the condition of the lubricating oil L and the condition of the bearing 18 based on the combination of the color of the reflected light and the color of the transmitted light. Therefore, the condition of the lubricating oil L and the condition of the bearing 18 can be monitored for maintenance of the vacuum pump 1 at an appropriate time.

In one embodiment, the data processor 40 calculates a color difference of the reflected light with respect to a reflected reference color based on the intensities of the red component, the green component, and the blue component of the reflected light, and calculates a color difference of the transmitted light with respect to a transmitted reference color based on the intensities of the red component, the green component, and the blue component of the transmitted light. The data processor 40 then judges the condition of the lubricating oil L and the condition of the bearing 18 based on the color difference of the reflected light and the color difference of the transmitted light. The reflected reference color may be a specific color (e.g., white), or may be a color of reflected light when the inspection light irradiates normal (new) lubricating oil. The transmitted reference color may be a specific color (e.g., white), or may be a color of transmitted light when the inspection light irradiates the normal (new) lubricating oil.

The color difference ΔE1_{RGB} of the reflected light with respect to the reflected reference color is expressed by the following formula (1) $ΔE {1}_{RGB} = \sqrt{{\left(R1a-R1x\right)}^{2} + {\left(G1a-G1x\right)}^{2} + {\left(B1a-B1x\right)}^{2}}$ (1) where R1a is an intensity of a red component of the reflected reference color, G1a is an intensity of a green component of the reflected reference color, B1a is an intensity of a blue component of the reflected reference color, R1x is the intensity of the red component of the reflected light from the lubricating oil L, G1x is the intensity of the green component of the reflected light from the lubricating oil L, and B1x is the intensity of the blue component of the reflected light from the lubricating oil L.

The color difference ΔE2_{RGB} of the transmitted light with respect to the transmitted reference color is expressed by the following formula (2) $ΔE {2}_{RGB} = \sqrt{{\left(R2a-R2x\right)}^{2} + {\left(G2a-G2x\right)}^{2} + {\left(B2a-B2x\right)}^{2}}$ (2) where R2a is an intensity of a red component of the transmitted reference color, G2a is an intensity of a green component of the transmitted reference color, B2a is an intensity of a blue component of the transmitted reference color, R2x is the intensity of the red component of the transmitted light from the lubricating oil L, G2x is the intensity of the green component of the transmitted light from the lubricating oil L, and B2x is the intensity of the blue component of the transmitted light from the lubricating oil L.

In one example, R1a, G1a, and B1a of the reflected reference color may be intensities of a red component, a green component, and a blue component of reflected light from normal (new) lubricating oil when irradiated with the inspection light. R2a, G2a, and B2a of the transmitted reference color may be intensities of a red component, a green component, and a blue component of transmitted light from normal (new) lubricating oil when irradiated with the inspection light.

The data processor 40 judges the condition of the lubricating oil L and the condition of the bearing 18 based on the color difference ΔE1_{RGB} of the reflected light and the color difference ΔE2_{RGB} of the transmitted light. The color difference ΔE1_{RGB} of the reflected light increases as a hue of the reflected light is deviated from a hue of the reflected reference color. Similarly, the color difference ΔE2_{RGB} of the transmitted light increases as a hue of the transmitted light is deviated from a hue of the transmitted reference color. For example, the data processor 40 judges that an abnormality has occurred in the bearing 18 or the lubricating oil L when the color difference ΔE1_{RGB} of the reflected light is larger than a predetermined reflected-color-difference threshold value and the color difference ΔE2_{RGB} of the transmitted light is larger than a predetermined transmitted-color-difference threshold value. The reflected-color-difference threshold value, the transmitted-color-difference threshold value, and criteria for judgment of the condition of the lubricating oil L and the condition of the bearing 18 are determined in advance based on the reflected reference color and the transmitted reference color, etc.

As shown in FIG. 1, the vacuum pump system further includes a pump controller 50 configured to control the operations of the vacuum pump 1, and a display device 55 configured to display an alarm on the condition of the lubricating oil L and the condition of the bearing 18. The data processor 40 and the display device 55 are electrically coupled to the pump controller 50. Judgement results of the condition of the lubricating oil L and the condition of the bearing 18 judged by the data processor 40 are transmitted to the pump controller 50. The pump controller 50 is configured to output an alarm signal when the data processor 40 judges that an abnormality has occurred in the bearing 18, when the data processor 40 judges that the lubricating oil L has been contaminated with foreign matter, or when the data processor 40 judges that the lubricating oil L has been deteriorated.

The display device 55 is constituted of a liquid crystal display or the like. The alarm signal output from the pump controller 50 is transmitted to the display device 55. The display device 55 displays the alarm on the condition of the lubricating oil L and the condition of the bearing 18 based on the alarm signal output from the pump controller 50. Therefore, an operator can perform maintenance of the vacuum pump 1 at an appropriate time based on the alarm displayed on the display device 55.

In one embodiment, when the data processor 40 judges that an abnormality has occurred in the bearing 18, the pump controller 50 may output a bearing-abnormality alarm signal indicating that an abnormality has occurred in the bearing 18. Similarly, when the data processor 40 judges that the lubricating oil L has been contaminated with foreign matter, the pump controller 50 may output a lubricating-oil-contamination alarm signal indicating that the lubricating oil L has been contaminated with foreign matter. When the data processor 40 judges that the lubricating oil L has been deteriorated, the pump controller 50 may output a lubricating-oil-deterioration alarm signal indicating that the lubricating oil L has been deteriorated.

In this case, the display device 55 displays an alarm indicating that an abnormality has occurred in the bearing 18 based on the bearing-abnormality alarm signal. Similarly, the display device 55 displays an alarm indicating that the lubricating oil L has been contaminated with foreign matter based on the lubricating-oil-contamination alarm signal, and displays an alarm indicating that the lubricating oil L has been deteriorated based on the lubricating-oil-deterioration alarm signal.

In one embodiment, the alarm indicating that the abnormality has occurred in the bearing 18 displayed on the display device 55 may include a message for urging a replacement of the bearing 18. The alarm indicating that the lubricating oil L has been contaminated with the foreign matter and the alarm indicating that the lubricating oil L has been deteriorated may include a message for urging a replacement of the lubricating oil L. Therefore, the operator can perform maintenance of the vacuum pump 1 at an appropriate time based on the message(s) displayed on the display device 55.

The pump controller 50 is composed of at least one computer. The pump controller 50 includes a memory 50a storing programs therein, and an arithmetic device 50b configured to perform arithmetic operations according to instructions contained in the programs. The memory 50a includes a main memory, such as a random access memory (RAM), and an auxiliary memory, such as a hard disk drive (HDD) or a solid state drive (SSD). Examples of the arithmetic device 50b include a CPU (central processing unit) and a GPU (graphics processing unit). However, the specific configurations of the pump controller 50 are not limited to these examples.

The previous description of embodiments is provided to enable a person skilled in the art to make and use the present invention. Moreover, various modifications to these embodiments will be readily apparent to those skilled in the art, and the generic principles and specific examples defined herein may be applied to other embodiments. Therefore, the present invention is not intended to be limited to the embodiments described herein but is to be accorded the widest scope as defined by limitation of the claims.

## Claims

1. A lubricating-oil monitoring (30) system for monitoring lubricating oil supplied to a bearing of a vacuum pump:
a lubricating-oil measuring device (31) configured to optically measure the lubricating oil supplied to the bearing (18) of the vacuum pump (1); and
a data processor (40) electrically coupled to the lubricating-oil measuring device (31),
wherein the lubricating-oil measuring device (31) further includes:
an oil inspection chamber (33) into which the lubricating oil from a lubricating-oil chamber (26) holding the lubricating oil is introduced;
a light source (35) configured to irradiate the lubricating oil in the oil inspection chamber (33) with inspection light containing red light, green light, and blue light; and
the data processor (40) is configured to judge a condition of the lubricating oil and a condition of the bearing (18) based on a combination of a color of the reflected light and a color of the transmitted light,
**characterised by**
a first light receiving element (36) configured to receive reflected light from the lubricating oil irradiated with the inspection light; and a second light receiving element (37) configured to receive transmitted light from the lubricating oil irradiated with the inspection light.

2. The lubricating-oil monitoring system (30) according to claim 1, wherein the data processor (40) is configured to:
resolve each of the reflected light and the transmitted light into a red component, a green component, and a blue component; and
judge the condition of the lubricating oil and the condition of the bearing (18) based on intensities of the red component, the green component, and the blue component of each of the reflected light and the transmitted light.

3. The lubricating-oil monitoring system (30) according to claim 2, wherein the condition of the lubricating oil and the condition of the bearing (18) include an abnormality in the bearing (18), contamination of the lubricating oil, and deterioration of the lubricating oil.

4. The lubricating-oil monitoring system (30) according to claim 3, wherein the data processor (40) is configured to judge that an abnormality has occurred in the bearing (18) when the intensities of the red component, the green component, and the blue component of the reflected light are smaller than a predetermined bearing-abnormality threshold value and the intensities of the red component, the green component, and the blue component of the transmitted light are smaller than the predetermined bearing-abnormality threshold value.

5. The lubricating-oil monitoring system (30) according to claim 3, wherein the data processor (40) is configured to judge that the lubricating oil has been contaminated with foreign matter when the intensities of the red component, the green component, and the blue component of the reflected light are larger than a predetermined lubricating-oil-contamination threshold value and the intensities of the red component, the green component, and the blue component of the transmitted light are smaller than the predetermined lubricating-oil-contamination threshold value.

6. The lubricating-oil monitoring system (30) according to claim 3, wherein the data processor (40) is configured to judge that the lubricating oil has been deteriorated when the intensity of the red component of the reflected light is larger than a predetermined lubricating-oil-deterioration threshold value and the intensities of the green component and the blue component of the reflected light are smaller than the lubricating-oil-deterioration threshold value.

7. The lubricating-oil monitoring system (30) according to claim 1, wherein the data processor (40) is configured to:
calculate a color difference of the reflected light with respect to a reflected reference color based on the intensities of the red component, the green component, and the blue component of the reflected light;
calculate a color difference of the transmitted light with respect to a transmitted reference color based on the intensities of the red component, the green component, and the blue component of the transmitted light; and
judge the condition of the lubricating oil and the condition of the bearing (18) based on the color difference of the reflected light and the color difference of the transmitted light.

8. The lubricating-oil monitoring system (30) according to claim 1, wherein the lubricating oil comprises a fluorine-based lubricating oil.

9. A vacuum pump system comprising:
a vacuum pump (1); and
the lubricating-oil monitoring system according to any one of claims 1 to 8,
wherein the vacuum pump (1) further includes:
the bearing (18); and
a housing structure (24) that forms a lubricating-oil chamber (26) holding the lubricating oil,
the lubricating-oil measuring device (31) is attached to the housing structure (24), and
the oil inspection chamber (33) communicates with the lubricating-oil chamber (26).

10. The vacuum pump system according to claim 9, further comprising:
a pump controller (50) configured to control an operation of the vacuum pump (1),
wherein the pump controller (50) is configured to output an alarm signal when the data processor (40) judges that an abnormality has occurred in the bearing (18), when the data processor (40) judges that the lubricating oil has been contaminated with foreign matter, or when the data processor (40) judges that the lubricating oil has been deteriorated.

## Patentansprüche

1. Ein Schmieröl-Überwachungs-System (30) zur Überwachung von Schmieröl, das einem Lager einer Vakuumpumpe zugeführt wird:
eine Schmieröl-Messeinrichtung (31), eingerichtet zum optischen Messen des Schmieröls, das dem Lager (18) der Vakuumpumpe (1) zugeführt wird; und
ein Datenprozessor (40), elektrisch mit der Schmieröl-Messeinrichtung (31) gekoppelt,
wobei die Schmieröl-Messeinrichtung (31) ferner Folgendes aufweist:
eine Öl-Prüfkammer (33), in die das Schmieröl aus einer Schmieröl-Kammer (26), die das Schmieröl hält, eingebracht wird;
eine Lichtquelle (35), eingerichtet zum Bestrahlen des Schmieröls in der Öl-Prüfkammer (33) mit Prüflicht, das rotes Licht, grünes Licht und blaues Licht enthält; und
der Datenprozessor (40) ist eingerichtet zum Beurteilen eines Zustands des Schmieröls und eines Zustands des Lagers (18) basierend auf einer Kombination einer Farbe des reflektierten Lichts und einer Farbe des transmittierten Lichts,
**gekennzeichnet durch**
ein erstes Lichtempfangselement (36), eingerichtet zum Empfangen von reflektiertem Licht von dem mit dem Prüflicht bestrahlten Schmieröl; und
ein zweites Lichtempfangselement (37), eingerichtet zum Empfangen von transmittiertem Licht von dem mit dem Prüflicht bestrahlten Schmieröl.

2. Das Schmieröl-Überwachungs-System (30) nach Anspruch 1, wobei der Datenprozessor (40) eingerichtet ist zum:
Zerlegen jeweils des reflektierten Lichts und des transmittierten Lichts in eine Rot-Komponente, eine Grün-Komponente und eine Blau-Komponente; und
Beurteilen des Zustands des Schmieröls und des Zustands des Lagers (18) basierend auf Intensitäten der Rot-Komponente, der Grün-Komponente und der Blau-Komponente jeweils des reflektierten Lichts und des transmittierten Lichts.

3. Das Schmieröl-Überwachungs-System (30) nach Anspruch 2, wobei der Zustand des Schmieröls und der Zustand des Lagers (18) eine Anomalie in dem Lager (18), eine Verunreinigung des Schmieröls und eine Verschlechterung des Schmieröls umfassen.

4. Das Schmieröl-Überwachungs-System (30) nach Anspruch 3, wobei der Datenprozessor (40) eingerichtet ist zum Beurteilen, dass eine Anomalie in dem Lager (18) aufgetreten ist, wenn die Intensitäten der Rot-Komponente, der Grün-Komponente und der Blau-Komponente des reflektierten Lichts kleiner als ein vorbestimmter Lager-Anomalie-Schwellenwert sind und die Intensitäten der Rot-Komponente, der Grün-Komponente und der Blau-Komponente des transmittierten Lichts kleiner als der vorbestimmte Lager-Anomalie-Schwellenwert sind.

5. Das Schmieröl-Überwachungs-System (30) nach Anspruch 3, wobei der Datenprozessor (40) eingerichtet ist zum Beurteilen, dass das Schmieröl mit Fremdmaterial verunreinigt worden ist, wenn die Intensitäten der Rot-Komponente, der Grün-Komponente und der Blau-Komponente des reflektierten Lichts größer als ein vorbestimmter Schmieröl-Verunreinigungs-Schwellenwert sind und die Intensitäten der Rot-Komponente, der Grün-Komponente und der Blau-Komponente des transmittierten Lichts kleiner als der vorbestimmte Schmieröl-Verunreinigungs-Schwellenwert sind.

6. Das Schmieröl-Überwachungs-System (30) nach Anspruch 3, wobei der Datenprozessor (40) eingerichtet ist zum Beurteilen, dass das Schmieröl verschlechtert worden ist, wenn die Intensität der Rot-Komponente des reflektierten Lichts größer als ein vorbestimmter Schmieröl-Verschlechterungs-Schwellenwert ist und die Intensitäten der Grün-Komponente und der Blau-Komponente des reflektierten Lichts kleiner als der Schmieröl-Verschlechterungs-Schwellenwert sind.

7. Das Schmieröl-Überwachungs-System (30) nach Anspruch 1, wobei der Datenprozessor (40) eingerichtet ist zum:
Berechnen einer Farbdifferenz des reflektierten Lichts in Bezug auf eine reflektierte Referenzfarbe basierend auf den Intensitäten der Rot-Komponente, der Grün-Komponente und der Blau-Komponente des reflektierten Lichts;
Berechnen einer Farbdifferenz des transmittierten Lichts in Bezug auf eine transmittierte Referenzfarbe basierend auf den Intensitäten der Rot-Komponente, der Grün-Komponente und der Blau-Komponente des transmittierten Lichts; und
Beurteilen des Zustands des Schmieröls und des Zustands des Lagers (18) basierend auf der Farbdifferenz des reflektierten Lichts und der Farbdifferenz des transmittierten Lichts.

8. Das Schmieröl-Überwachungs-System (30) nach Anspruch 1, wobei das Schmieröl ein Fluor-basiertes Schmieröl aufweist.

9. Ein Vakuumpumpen-System, das Folgendes aufweist:
eine Vakuumpumpe (1); und
das Schmieröl-Überwachungs-System nach einem der Ansprüche 1 bis 8, wobei die Vakuumpumpe (1) ferner Folgendes aufweist:
das Lager (18); und
eine Gehäusestruktur (24), die eine Schmieröl-Kammer (26) ausbildet, die das Schmieröl hält,
die Schmieröl-Messeinrichtung (31) an der Gehäusestruktur (24) befestigt ist, und
die Öl-Prüfkammer (33) mit der Schmieröl-Kammer (26) in Verbindung steht.

10. Das Vakuumpumpen-System nach Anspruch 9, ferner aufweisend:
eine Pumpen-Steuereinrichtung (50), eingerichtet zum Steuern eines Betriebs der Vakuumpumpe (1),
wobei die Pumpen-Steuereinrichtung (50) eingerichtet ist zum Ausgeben eines Alarmsignals, wenn der Datenprozessor (40) beurteilt, dass eine Anomalie in dem Lager (18) aufgetreten ist, wenn der Datenprozessor (40) beurteilt, dass das Schmieröl mit Fremdmaterial verunreinigt worden ist, oder wenn der Datenprozessor (40) beurteilt, dass das Schmieröl verschlechtert worden ist.

## Revendications

1. Système de surveillance d'une huile de lubrification (30) destiné à surveiller une huile de lubrification fournie à un roulement d'une pompe à vide :
un dispositif de mesure de l'huile de lubrification (31) configuré pour mesurer optiquement l'huile de lubrification fournie au roulement (18) de la pompe à vide (1) ; et
un processeur de données (40) couplé électriquement au dispositif de mesure de l'huile de lubrification (31),
dans lequel le dispositif de mesure de l'huile de lubrification (31) comporte en outre :
une chambre d'inspection de l'huile (33) dans laquelle l'huile de lubrification est introduite à partir d'une chambre à huile de lubrification (26) contenant l'huile de lubrification ;
une source de lumière (35) configurée pour irradier l'huile de lubrification dans la chambre d'inspection de l'huile (33) avec une lumière d'inspection contenant de la lumière rouge, de la lumière verte et de la lumière bleue ; et
le processeur de données (40) est configuré pour évaluer un état de l'huile de lubrification et un état du roulement (18) sur la base d'une combinaison d'une couleur de la lumière réfléchie et d'une couleur de la lumière transmise,
**caractérisé par**
un premier élément récepteur de lumière (36) configuré pour recevoir la lumière réfléchie par l'huile de lubrification irradiée avec la lumière d'inspection ; et un deuxième élément récepteur de lumière (37) configuré pour recevoir la lumière transmise par l'huile de lubrification irradiée avec la lumière d'inspection.

2. Système de surveillance d'une huile de lubrification (30) selon la revendication 1, dans lequel le processeur de données (40) est configuré pour :
décomposer chacune parmi la lumière réfléchie et la lumière transmise en une composante rouge, une composante verte et une composante bleue ; et
évaluer l'état de l'huile de lubrification et l'état du roulement (18) sur la base des intensités de la composante rouge, de la composante verte et de la composante bleue de la lumière réfléchie et de la lumière transmise.

3. Système de surveillance d'une huile de lubrification (30) selon la revendication 2, dans lequel l'état de l'huile de lubrification et l'état du roulement (18) comportent une anomalie du roulement (18), une contamination de l'huile de lubrification et une détérioration de l'huile de lubrification.

4. Système de surveillance d'une huile de lubrification (30) selon la revendication 3, dans lequel le processeur de données (40) est configuré pour évaluer qu'une anomalie s'est produite dans le roulement (18) lorsque les intensités de la composante rouge, de la composante verte et de la composante bleue de la lumière réfléchie sont inférieures à une valeur seuil prédéterminée d'anomalie du roulement et que les intensités de la composante rouge, de la composante verte et de la composante bleue de la lumière transmise sont inférieures à la valeur seuil prédéterminée d'anomalie du roulement.

5. Système de surveillance d'une huile de lubrification (30) selon la revendication 3, dans lequel le processeur de données (40) est configuré pour évaluer que l'huile de lubrification a été contaminée par des matières étrangères lorsque les intensités de la composante rouge, de la composante verte et de la composante bleue de la lumière réfléchie sont supérieures à une valeur seuil prédéterminée de contamination de l'huile de lubrification et que les intensités de la composante rouge, de la composante verte et de la composante bleue de la lumière transmise sont inférieures à la valeur seuil prédéterminée de contamination de l'huile de lubrification.

6. Système de surveillance d'une huile de lubrification (30) selon la revendication 3, dans lequel le processeur de données (40) est configuré pour évaluer que l'huile de lubrification s'est détériorée lorsque l'intensité de la composante rouge de la lumière réfléchie est supérieure à une valeur seuil prédéterminée de détérioration de l'huile de lubrification et que les intensités de la composante verte et de la composante bleue de la lumière réfléchie sont inférieures à la valeur seuil de détérioration de l'huile de lubrification.

7. Système de surveillance d'une huile de lubrification (30) selon la revendication 1, dans lequel le processeur de données (40) est configuré pour :
calculer une différence de couleur de la lumière réfléchie par rapport à une couleur de référence réfléchie sur la base des intensités de la composante rouge, de la composante verte et de la composante bleue de la lumière réfléchie ;
calculer une différence de couleur de la lumière transmise par rapport à une couleur de référence transmise, sur la base des intensités de la composante rouge, de la composante verte et de la composante bleue de la lumière transmise ; et
évaluer l'état de l'huile de lubrification et l'état du roulement (18) sur la base de la différence de couleur de la lumière réfléchie et de la différence de couleur de la lumière transmise.

8. Système de surveillance d'une huile de lubrification (30) selon la revendication 1, dans lequel l'huile de lubrification comprend une huile de lubrification à base de fluor.

9. Système de pompe à vide comprenant :
une pompe à vide (1) ; et
le système de surveillance de l'huile de lubrification selon l'une quelconque des revendications 1 à 8,
dans lequel la pompe à vide (1) comporte en outre :
le roulement (18) ; et
une structure de boîtier (24) qui constitue une chambre à huile de lubrification (26) contenant l'huile de lubrification,
le dispositif de mesure de l'huile de lubrification (31) est fixé à la structure du boîtier (24), et
la chambre d'inspection de l'huile (33) communique avec la chambre à huile de lubrification (26).

10. Système de pompe à vide selon la revendication 9, comprenant en outre :
un contrôleur de pompe (50) configuré pour contrôler le fonctionnement de la pompe à vide (1),
dans lequel le contrôleur de pompe (50) est configuré pour fournir en sortie un signal d'alarme lorsque le processeur de données (40) évalue qu'une anomalie s'est produite au niveau du roulement (18), lorsque le processeur de données (40) évalue que l'huile de lubrification a été contaminée par des corps étrangers, ou lorsque le processeur de données (40) évalue que l'huile de lubrification s'est détériorée.
